# EUROPEAN PATENT SPECIFICATION

(11) **EP 0 705 344 B1**
(45) Date of publication and mention of the grant of the patent: **14.09.2005**
(21) Application number: 94920351.7
(22) Date of filing: 24.06.1994
(51) Int. Cl.: C12N 15/861, C12N 15/00

(54) **ADENOVIRUS VECTORS FOR GENE THERAPY**
ADENOVIRUS VEKTOREN FÜR GENTHERAPIE
VECTEURS A BASE D'ADENOVIRUS DESTINES A LA THERAPIE GENIQUE

(30) Priority: 24.06.1993 US 80727; 31.05.1994 US 250885
(43) Date of publication of application: 10.04.1996
(73) Proprietor: Graham, Frank L., Ontario L8P 2V4 (CA); Bett, Andrew, Ontario L8S 4H6 (CA); Haddara, Wael, Ontario L8S 1A1 (CA); Prevek, Ludvik, Ontario L7T 1M9 (CA)
(72) Inventor: Graham, Frank L., Ontario L8P 2V4 (CA); Bett, Andrew, Ontario L8S 4H6 (CA); Haddara, Wael, Ontario L8S 1A1 (CA); Prevek, Ludvik, Ontario L7T 1M9 (CA)
(74) Representative: Germinario, Claudio
(86) International application number: PCT/CA1994/000364
(87) International publication number: WO 1995/000655

(56) References cited:
- WO-A-93/06223
- WO-A-93/19092
- WO-A-93/19191
- WO-A-94/08026
- WO-A-94/12649
- PROCEEDINGS OF THE NATIONAL ACADEMY OF SCIENCES OF USA., vol.89, no.7, 1 April 1992, WASHINGTON US pages 2581 - 2584 QUANTIN ET AL. 'Adenovirus as an expressionvector in muscle cells in vivo'
- CELL., vol.68, no.1, 10 January 1992, CAMBRIDGE, NA US pages 143 - 155 ROSENFELD ET AL. 'In vivo transfer of the human cystic fibrosis transmembrane conductance regulator gene to the airway epithelium'
- JOURNAL OF VIROLOGY, vol. 61, no. 8, August 1987 (1987-08), pages 2555-2558,

## Description

### FIELD OF THE INVENTION

This invention relates to adenovirus (Ad) vectors that are useful for enhanced expression of selected nucleic acids in infected, transfected or transformed cells, especially eukaryotic mammalian cells. This invention also generally relates to genetically engineered vectors that encode therapeutic substances useful as vaccines and for gene therapy.

### BACKGROUND

Adenoviruses (Ads) are a relatively well characterized, homogeneous group of viruses. Roughly 100 different adenoviruses, including nearly 50 serotypes isolated from humans, have been identified to date.

Most common serotypes of Ads are nonpathogenic, physically and genetically stable, can be grown to very high titres (concentrated stocks with 10¹¹-10¹² PFU/ml of infectious virus are easy to obtain) and easily purified by isopycnic centrifugation in CsCl gradients. The Ad genome is readily manipulated by recombinant DNA techniques, and the proteins encoded by foreign DNA inserts that are expressed in mammalian cells will usually be appropriately glycosylated or phosphorylated, unlike recombinant proteins expressed in bacteria, yeast, and some insect cells. Although human Ads replicate most efficiently in human cells of epithelial origin, these viruses infect almost any mammalian cell and express at least some viral genes. Unlike retroviruses, Ads will infect, and are expressed in, nonreplicating cells. Thus, Ad-based vectors may be useful for gene delivery, expression, and gene therapy.

Ad vectors have been constructed by ligation or recombination of viral DNA with subgenomic viral sequences contained in bacterial plasmids. Berkner, K.L. and Sharp, P.A., 1983, *Nucleic Acids Res.* 11: 6003-6020; Haj-Ahmad, Y. and Graham, F.L., 1986, *J. Virol*. 57: 267-274; Stow, N.D., 1981, *J. Virol*. 37: 171-180. This approach has several drawbacks, which include the time and technical difficultly required to produce viral DNA, the background of infectious parental virus which makes screening more labor intensive and, in the case of direct ligation, the limited availability of useful restriction sites due to the relatively large size of the adenovirus genome.

Another strategy has been to recombine two plasmids which together contain sequences comprising the entire Ad genome. A number of conditionally defective plasmid systems have been developed making the construction of vectors simpler and reducing the number of subsequent analyses required to identify recombinant viruses. McGrory, W.J., Bautista, D.S. and Graham, F.L., 1988, *Virol*. 163: 614-617; Ghosh-Choudhury, G., Haj-Ahmad, Y., Brinkley, P., Rudy, J. and Graham, F.L., 1986, *Gene* 50: 161-171; Mittal, S.K., McDermott, M.R. Johnson, D.C., Prevec, L. and Graham, F.L., 1993, Virus *Res. 28:* 67-90.

The representative Adenovirus 5 ("Ad5") genome used in embodiments of the present invention is a 36kb linear duplex. Its sequence has been published. (Chroboczek, J., Bieber, F., and Jacrot, B., 1992, The Sequence of the Genome of Adenovirus Type 5 and Its Comparison with the Genome of Adenovirus Type 2, *Virol*. 186: 280-285; hereby incorporated by reference). The Ad5 genome contains a 100-150 base pair (bp) inverted terminal repeat ("ITR") at each end of the linearized genome. A terminal protein ("TP") of 55,000 daltons is covalently linked to the 5' end of each strand. Both the TP and the ITRs are thought to play a role in viral DNA replication. McGrory, W. J. *et al*., 1988, *Virol*. 163: 614-617 and Ghosh-Choudhury, G. *et al*., 1986, *Gene* 50: 161-171.) Ad5 has infected each human cell line tested, although some cells, such as lymphocytes, are relatively nonpermissive.

Four noncontiguous regions of the Ad5 genome are transcribed early in infection, prior to DNA replication. These regions are early region 1 (E1) (about 1.3-11.2 mu of or about position 198-4025 bp of a standardized genome, inclusive of the E1A enhancer region; Sussenbach, J. S., 1984, in Ginsburg (Ed.), THE ADENOVIRUSES, Plenum Press, pp. 35-124) which is further divided into subregions E1A and E1B; early region 2 (E2), which encodes the DNA replicative functions of the virus; early region 3 (E3) (about 75.9-86.0 mU, or about 27,275-30,904 bp; Cladaras, C. and Wold, W.S.M., 1985, *Virol*. 140: 28-43; and early region 4 (E4). E1A is involved in turning on the other early regions and in regulating a number of host cell functions. E1B and E4 are primarily involved in shutting off the host cell's protein synthesis. E3 regulates the host cell's immune response to virus infection. Some of these early genes function to "turn on" later-expressed genes that are needed to replicate the genome and form viral particles.

Various Ad vectors have been described. For example, an Ad5 vector has been constructed from an adenovirus DNA fragment and a linearized plasmid. Quantin et al., 1992, Proc. Natl. Acad. Sci. USA 89:2581-2584. A recombinant adenovirus vector constructed from CTFR expression cassette and an adenovirus DNA fragment has been described. Rosenfeld et al., 1992, Cell 68:143-155.

The Ad virion has the ability to package up to 105-106% of the wild type genome length. Bett, A.J., Prevec, L., and Graham, F.L., 1993, Packaging Capacity and Stability of Human Adenovirus Type 5 Vectors, J. Virol. 67:5911-5921. Larger genomes (e.g., 108% of the wild type in size), result in instability of the virus and poor growth rates. *Id*. This packaging ability allows the insertion of only approximately 1.8-2.0 kb of excess DNA into the Ad genome.

To package larger inserts, it is necessary to first delete portions of the viral genome. Parts of region E1 can be deleted, and the resulting viruses can be propagated in human 293 cells. (293 cells contain and express E1, complementing viral mutants that are defective in E1.) Foreign nucleic acids can be inserted in place of E1, in Ad5 genomes that contain E1 deletions of up to 2.9 kb, to yield conditional helper-independent vectors with a capacity for inserts of 4.7-4.9 kb.

Viruses with a region E3 deletion can also replicate in cultured human cells such as HeLa or KB and infect and be expressed in animals including humans. A deletion of a 3.0 kb E3 sequence has been reported, without a concomitant insertion. Ranheim, T.S., Shisler, J., Horton, T.M., Wold, L.J. Gooding, L.R., and Wold, W.S.M., 1993, J. Virol. 67:2159-2167.

Among the methods developed to date there is no simple procedure for generating vectors that utilize both E1 and E3 deletions. In addition, the vectors that do utilize either E1 or E3 deletions can accommodate only relatively small inserts. To simplify the production and use of Ad vectors that can tolerate larger fragments, we have developed a new methodology based on a series of bacterial plasmids that contain most of an Ad viral genome.

### SUMMARY OF THE INVENTION

It is a goal of this invention to provide simple, flexible, efficient, high capacity Ad 5 cloning and expression vectors. Accordingly, a new vector system has been developed which comprises expanded deletions in both E1 and E3 and further combines them in a single vector system that can tolerate inserts of up to 8000 bp of inserts, enough to accommodate the majority of protein coding genes along with control elements to regulate expression. The invention provides the option of cloning foreign nucleic acids into either or both of the E1 or E3 regions and promises to be the most versatile and easy to use technology yet developed. In addition, a modification of the system permits construction of viruses carrying a wild type E3 region, and insertions, substitutions, or mutations in the E1 region.

One embodiment of the present invention provides a bacterial plasmid comprising a circularized modified human adenovirus type 5 (Ad5) genome. The nucleotide sequence of the plasmid has a deletion within early region 3 (E3) of said Ad5 genome, and a segment of bacterially replicable pBR322 plasmid encoding ampicillin resistance substituted for a sequence of early region 1A (E1A) that corresponds, in whole or in part, to the packaging signal.

Another embodiment provides a bacterial plasmid comprising approximately 340 base pairs from the left end of the adenovirus type 5 genome, the left end inverted terminal repeat sequences of said genome and the packaging signal sequences thereof, said plasmid comprising also a eukaryotic gene sequence of up to about 8 kilobases foreign to said plasmid and to said viral genome. The adenovirus sequence from approximately nucleotide position 3540 thereof to approximately position 5790 thereof is present on the right side of said foreign sequence.

Other embodiments of the present invention include adenovirus genome constructs containing E1 deletions and foreign inserts of eukaryotic origin, using any combination of size of E1 deletion and/or of size of foreign insert that can be accommodated in the plasmid and still remain operable. Because of the large capacity of the vectors provided herein, multiple inserts of foreign genes can be placed in the E1 cloning site. For example, two or more genes encoding different antigens, or genes encoding useful proteins, can be combined with genes encoding chemically selectable markers.

One specific embodiment of the invention, the plasmid pBHG10, may be used to insert foreign genes into either the E3 or E1 region of the Ad5 genome. Genes inserted into E3 can be combined with a variety of mutations, deletions, or insertions in E1 by appropriate choice of the cotransfected plasmid containing left end (E1) sequences.

### BRIEF DESCRIPTION OF THE DRAWINGS

**Fig. 1** is a diagrammatic representation of the structure and construction of the vector pBHG10.
**Fig. 2** is a diagrammatic representation of the structure and construction of the vector pBHG3.
**Fig. 3** is a diagrammatic representation of rescue using pBHG vectors.
**Fig. 4** is a diagrammatic representation of the structure and construction of a 3.2 kb E1 deletion, and two examples (pΔE1sp1A and pΔE1sp1B) of plasmids that contain said deletion.
**Fig. 5** illustrates the different levels of protein IX synthesized using plasmids having different E1 deletions with or without a reintroduced Ssp1 site.
**Fig. 6** illustrates heat stability of viruses with the 3.2 kb E1 deletion with or without a reintroduced Ssp1 site.
**Fig. 7** illustrates the construction and rescue of a 7.8 kb insert using pBGH10.
**Fig. 8** depicts the strategy for the construction of a double recombinant containing lacZ in the E3 deletion and firefly luciferase in the E1 deletion.
**Fig. 9** is a diagrammatic representation of the plasmids pABS.6, pABS.7, and pABS.9.
**Fig. 10** is a diagrammatic representation of the shuttle plasmids pHCMVsp1A, pHCMVsp1B, pHCMVsp1C, and pHCMVsp1D.

### DETAILED DESCRIPTION OF THE INVENTION

The recombinant Ad vectors provided herein are significantly different from previously reported constructs partly because they contain the largest possible deletion of E1 sequences (within 30-40 bp) that can be made while still allowing the generation of viable viral recombinants. Surprisingly, the different genetic elements described herein, when combined, produced a stable construct useful in introducing and expressing foreign nucleic acids in host cells.

At the onset of these experiments, it was unknown how large a deletion could be, or where it could be placed, without affecting the growth, production and infectivity of packaged virions. For viral viability and maximum packaging capacity, deletions in the E1 region preferably should not affect the left inverted terminal repeat (ITR; 1-103 bp) or packaging signals (194-358 bp). Hearing, P. and Shenk, T., 1983, *Cell,* 33: 695-703; Grable, M. and Hearing, P., 1992, *J. Virol*. 64: 2047-2056. In addition, because the only currently available E1 complementing cell line (293 cells) does not express protein IX, deletions should not extend into the coding sequences for this polypeptide. (Although viral deletion mutants lacking the protein IX gene have been isolated, it appears that the protein is essential for packaging of full length genomes into functional virus.)

In the pBHG plasmid embodiments of the invention, the pBR322 sequences substitute for Ad5 sequences from position 188 to 1339, which include the packaging signal, E1A enhancer, promoter and most of the E1A protein coding sequences. The pBR322 insert not only contains an ampicillin resistance, but allows allows the pBHG family of vectors to be replicated in cells wherein pBR322 may be replicated.

Some embodiments of the invention herein contain a deletion of the E1 region between an Ssp I site at 339 bp and an Afl site at 3533 bp. Since the Ssp1 site may be essential for protein IX expression, it was reintroduced as a synthetic oligonucleotide which positioned the SspI site closer to the protein IX TATA box than is the case in the wild type (wt) protein IX gene.

### Definitions

All technical and scientific terms used herein, unless otherwise defined, are generally intended to have the same meaning as commonly understood by one of ordinary skill in the art. A number of the terms used herein are not intended to be limiting, even though common usage might suggest otherwise. For example, the term "expression of" or "expressing" a foreign nucleic acid, gene or cDNA is used hereinafter to encompass the replication of a nucleic acid, the transcription of DNA and/or the translation of RNA into protein, in cells or in cell-free systems such as wheat germ or rabbit reticulocytes; and "nucleic acid" is used interchangeably with gene, cDNAs, RNA, or other oligonucleotides that encode gene products. The term "foreign" indicates that the nucleic acid is not found in nature identically associated with the same vector or host cell, but rather that the precise association between said nucleic acid and the vector or host cell is created by genetic engineering. The terms "recombinant" and "recombination" generally refer to rearrangements of genetic material that are contemplated by the inventors, and that are the result of experimental manipulation.

"Vector" denotes a genetically engineered nucleic acid construct capable of being modified by genetic recombinant techniques to incorporate any desired foreign nucleic acid sequence, which may be used as a means to introduce said sequence in a host cell, replicate it, clone it, and/or express said nucleic acid sequence, wherein said vector comprises all the necessary sequence information to enable the vector to be replicated in host cells, and/or to enable the nucleic acid sequence to be expressed, and/or to enable recombination to take place, and/or to enable the vector to be packaged in viral particles. This recitation of the properties of a vector is not meant to be exhaustive.

Vectors, optionally containing a foreign nucleic acid, may be "introduced" into a host cell, tissue or organism in accordance with known techniques, such as transformation, transfection using calcium phosphate-precipitated DNA, electroporation, gene guns, transfection with a recombinant virus or phagemid, infection with an infective viral particle, injection into tissues or microinjection of the DNA into cells or the like. Both prokaryotic and eukaryotic hosts may be employed, which may include bacteria, yeast, plants and animals, including human cells.

A vector "supports the expression of coding sequences contained by the vector" when it serves as a vehicle for the introduction of a gene into a host cell, when sequences present in the vector enable the vector and the coding regions that it contains to be replicated and to be maintained in a cell without being degraded, and when sequences present in the vector enable the coding sequences to be transcribed, recombined, or integrated into the host cell genome.

Once a given structural gene, cDNA or open reading frame has been introduced into the appropriate host, the host may be grown to express said structural gene, cDNA or open reading frame. Where the exogenous nucleic acid is to be expressed in a host which does not recognize the nucleic acid's naturally occurring transcriptional and translational regulatory regions, a variety of transcriptional regulatory regions may be inserted upstream or downstream from the coding region, some of which are externally inducible. Illustrative transcriptional regulatory regions or promoters for use in bacteria include the β-gal promoter, lambda left and right promoters, trp and lac promoters, trp-lac fusion promoter, and also the bacteriophage lambda P_{L} promoter together with the bacteriophage lambda O_{L} operator and the CI857 temperature-sensitive repressor, for example, to provide for temperature sensitive expression of a structural gene. Regulation of the promoter is achieved through interaction between the repressor and the operator. For use in yeast, illustrative transcriptional regulatory regions or promoters include glycolytic enzyme promoters, such as ADH-I and -II promoters, GPK promoter, and PGI promoter, TRP promoter, etc.; for use in mammalian cells, transcriptional control elements include SV40 early and late promoters, adenovirus major late promoters, etc. Other regulatory sequences useful in eucaryotic cells can include, for example, the cytomegalovirus enhancer sequence, which can be fused to a promoter sequence such as the SV40 promoter to form a chimeric promoter, or can be inserted elsewhere in the expression vehicle, preferably in close proximity to the promoter sequence. Where the promoter is inducible, permissive conditions may be employed (for example, temperature change, exhaustion, or excess of a metabolic product or nutrient, or the like).

When desired, expression of structural genes can be amplified by, for example, ligating in tandem a nucleic acid for a dominant amplifiable genetic marker 5' or 3' to the structural gene and growing the host cells under selective conditions. An example of an amplifiable nucleic acid is the gene for dihydrofolate reductase, expression of which may be increased in cells rendered resistant to methotrexate, a folate antagonist.

The expression vehicles used or provided herein may be included within a replication system for episomal maintenance in an appropriate cellular host, they may be provided without a replication system, or they may become integrated into the host genome.

While a wide variety of host cells are contemplated, certain embodiments require that the host cell express E1 sequences that are missing from or inactivated in the vector. While the human 293 cell line is the preferred host cell, the invention also contemplates other cell lines capable of complementing the vector having an E1 deletion. "Complementing" or "complemented by" denotes that the host cell line encodes and/or expresses functions that are necessary for generating viable viral particles that are missing from or have been inactivated in the vector.

It is important to recognize that the present invention is not limited to the use of such cells as are used herein. Cells from different species (human, mouse, etc.) or different tissues (breast epithelium, colon, neuronal tissue, lymphocytes, etc.) may also be used.

"Modification" of a nucleic acid includes all molecular alterations of a nucleic acid sequence that change its capacity to perform a stated function, specifically including deletions, insertions, chemical modifications, and the like. Insertions and deletions may be made in a number of ways known to those skilled in the art, including enzymatically cutting the full length sequence followed by modification and ligation of defined fragments, or by site-directed mutagenesis, especially by loop-out mutagenesis of the kind described by Kramer *et al*., 1984, *Nucl. Acids Res.* 12: 9441-9456.

"Fragment" refers to an isolated nucleic acid derived from a reference sequence by excising or deleting one or more nucleotides at any position of the reference sequence using known recombinant techniques, or by inserting a predetermined nucleotide or sequence of nucleotides at any predetermined position within the reference sequence using known recombinant techniques, or by substituting a predetermined nucleotide or sequence of nucleotides for a predetermined nucleotide or sequence of nucleotides within the reference sequence using known recombinant techniques. It is not intended that the invention be limited to the use of nucleic acid sequences from any particular species or genus, but that this invention can be carried out using nucleic acids from a variety of sources. It is contemplated that any nucleic acid from any source may be inserted into the vector, with or without control elements.

"Gene therapy" comprises the correction of genetic defects as well as the delivery and expression of selected nucleic acids in a short term treatment of a disease or pathological condition.

Reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, etc., such that a different but known way is used to achieve the same goals as those to which the use of a suggested method, material or composition is directed.

The present invention is not limited to the use of all of the described discoveries or embodiments explicitly described herein. Although combining them may indeed be preferred, it is not necessary to the invention that all aspects be used simultaneously.

The isolated nucleic acids of this invention can be used to generate modified polypeptides, each having at least one characteristic of the native polypeptide. These include subfragments, deletion mutants, processing mutants, or substitution mutants, polypeptides having the same secondary structure as the binding region of the native polypeptide, and combinations thereof. Such modified polypeptides may carry the functionality of the "wild type" peptide, or may have a modified or externally regulatable functionality. Such modified polypeptides may have considerable utility in the present invention, as would be appreciated by those skilled in the art.

"Wild type", mutant and analogous polypeptides and compositions thereof may be used for making antibodies, which may find use in analyzing results of the assays described as part of this invention. The antibodies may be prepared in conventional ways, either by using the subject polypeptide as an immunogen and injecting the polypeptide into a mammalian host, e.g., mouse, cow, goat, sheep, rabbit, etc., particularly with an adjuvant, e.g., complete Freund's adjuvant, aluminum hydroxide gel, or the like. The host may then be bled and the blood employed for isolation of polyclonal antibodies, or the peripheral blood lymphocytes (B-cells) may be fused with an appropriate myeloma cell to produce an immortalized cell line that secretes monoclonal antibodies specific for the subject compounds.

All publications mentioned herein are incorporated by reference.

### Enzymes, Cells and viruses

Enzymes used for recombinant DNA manipulations were purchased from Boehringer-Mannheim, Inc. (Laval, Quebec, Canada), New England Biolabs (Beverly, MA) or Bethesda Research Laboratories (Burlington, Ontario, Canada) and used according to the supplier's recommendations. Plasmids were constructed using standard protocols. Sambrook, J., E. F. Fritsch, and T. Maniatis, 1989, MOLECULAR CLONING: A LABORATORY MANUAL, 2nd Ed., Cold Spring Harbor Laboratory, Cold Spring Harbor, N.Y. Electroporation was used to transform *E. coli* strain DH5 (supE44 hsdR17 recA1 endA1 gyrA96 thi-1 relA1) with newly constructed plasmids. Dower, W. J., J. F. Miller, and C. W. Ragsdale, 1988, High efficiency transformation of *E. coli* by high voltage electroporation, *Nucleic Acids Res.* 16: 6127-6145. Plasmid DNA was prepared by the alkaline lysis method and purified by CsCl-Ethidium Bromide density gradient centrifugation. Birnboim, H.C., and J. Doly, 1978, A rapid alkaline extraction procedure for screening recombinant plasmid DNA, *Nucleic Acids Res.* 7: 1513-1523.

Cell culture media and reagents were obtained from GIBCO Laboratories (Grand Island, NY). Adenovirus (Ad) vectors were tittered and passaged on 293 cells which constitutively express the left 11% of the Ad5 genome, comprising the E1 region. Graham, F. L., J. Smiley, W. C. Russell, and R. Nairn, 1977, Characteristics of a human cell line transformed by DNA from human adenovirus type 5, *J. Gen. Virol*. 36: 59-72. The 293 cells were grown in monolayer in F-11 minimum essential medium supplemented with 100 units penicillin/ml, 100 µg streptomycin/ml, 2.5 µg amphotericin/ml and with 10% newborn calf serum for cell maintenance or 5% horse serum for virus infection. KB cells grown in spinner culture were maintained in Joklik's modified medium supplemented with antibiotics as above and with 10% horse serum.

For one step growth curves KB cells were grown to a density of 2x10⁵ cells/ml, centrifuged, and resuspended in 1/10th the volume of original medium and virus was added (20 PFU/cell) and allowed to adsorb for 1 h at 37°C with shaking. The cells were then returned to the original volume using 50% fresh and 50% original medium. At various times post-infection 4 ml aliquots were taken, 0.5 ml of glycerol added, and the samples were stored at -70°C for assays of infectious virus by plaque titration.

### Construction and growth of recombinant viruses

Recombinant viruses were isolated by cotransfection of 293 cells with appropriate plasmids. Graham, F. L., and A. J. Van der Eb, 1973, A new technique for the assay of infectivity of human adenovirus 5 DNA, *Virol*., 52: 456-467. After 8-10 days plaques were isolated, expanded and viral DNA analyzed by restriction enzyme digestion as described previously. Graham, F.L. and L. Prevec, 1991, Manipulation of Adenovirus Vectors, in E.J. Murry (ed.) METHODS IN MOLECULAR BIOLOGY, Vol. 7: GENE TRANSFER AND EXPRESSION PROTOCOLS, The Humana Press Inc., Clifton, N.J., p. 109-128. Candidate viruses were then plaque purified once and, for stability studies, vectors were passaged starting with medium from cells infected for viral DNA analysis after the first plaque purification. Semiconfluent monolayers of 293 cells in 60 mm dishes were infected with 0.5 ml of medium from each previous passage (approximately 40 PFU/cell), virus was allowed to adsorb for one half hour and then medium was replaced. Cells and medium were harvested when cytopathic effect was complete, usually within 2-3 days postinfection.

### ³²P labelling and extraction of viral DNA

Semiconfluent monolayers of 293 cells in 60 mm dishes were infected with virus from passages to be analyzed and at 24 h postinfection, medium was removed and replaced with 1 ml of phosphate-free 199 medium containing 5% horse serum and 25 uCi/ml of ³²P-orthophosphoric acid (purchased from DuPont de Nemours & Co., Inc., Wilmington, DE). After incubating the infected cells for a further 6 h, the cells were harvested and DNA was extracted. Viral DNA was then digested with appropriate restriction enzymes, electrophoresed through 1% agarose gels and the gels were dried and DNA bands visualized by autoradiography.

### Example 1: Generation of the plasmid pBHG10

Adenoviruses carry a cis-acting sequence in the E1 region which is essential for encapsidation of viral DNA molecules. When this cis-acting signal, located from 194 to 358 bp in Ad5, is deleted, viral genomes cannot be packaged but would still be expected to replicate their DNA in transfected cells. This, the fact that Ad DNA can circularize in infected cells, and that the cotransfection into mammalian cells of two plasmids with overlapping sequences can generate infectious virus with good efficiency, led us to conceive the strategy described below.

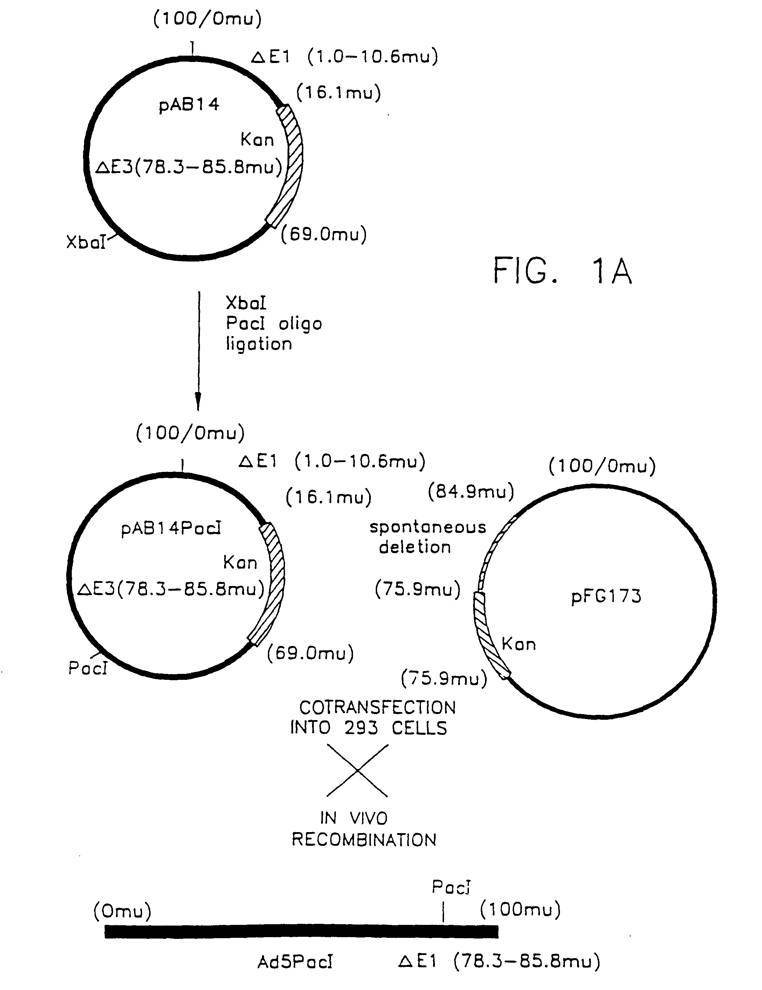

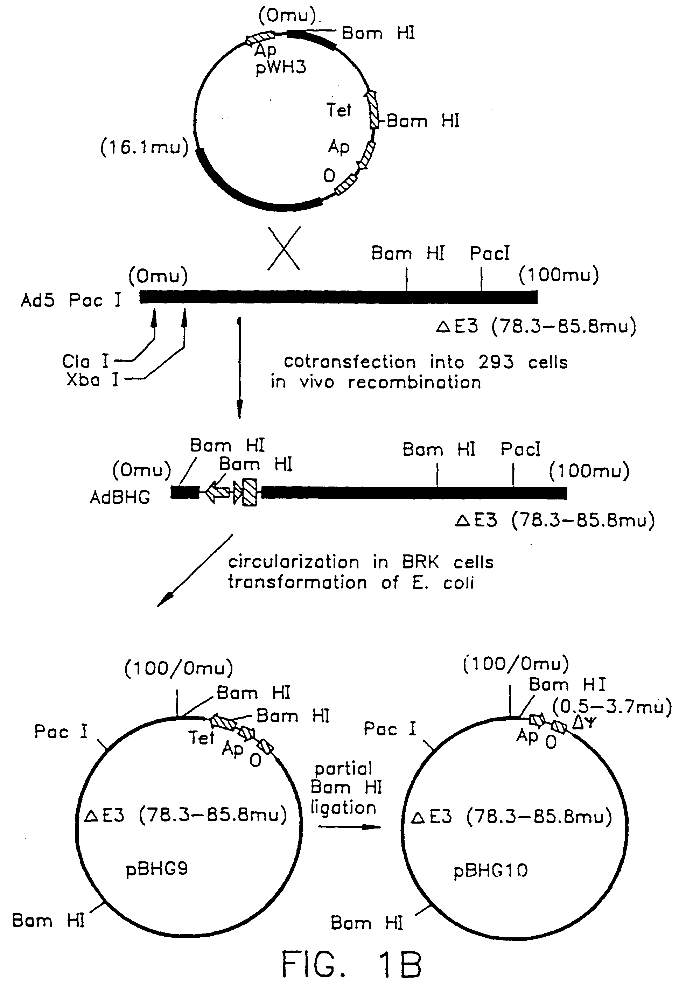

The first step involved the construction of Ad5PacI, a virus which contains the entire Ad5 genome with a deletion of E3 sequences from 28133 to 30818 bp. Ad5PacI was made by cotransfection of 293 cells with two plasmids: pFG173; and pAB14PacI, a modified pAB14 (Bett, A..J., L. Prevec, and Graham, F.L., 1993, *J. Virol*. 67: 5911-5921), in which a PacI cloning site is substituted in place of 2.69 kb of E3. (Fig. 1A). Next, purified viral DNA from Ad5PacI was digested with ClaI and XbaI and was cotransfected into 293 cells with another plasmid, pWH3 (Bautista, D.S., and Graham, F.L., 1989, *Gene* 82: 201-208), to yield the virus AdBHG (Fig. 1B). pWH3 is a plasmid containing left end Ad5 sequences, with an insertion of modified pBR322 plasmid at bp 1339, designed so that the packaging signals could be deleted at a later stage.

The next step involved the generation of a bacterial plasmid containing the entire AdBHG genome and subsequent identification of infectious clones. Baby rat kidney (BRK) cells were infected with AdBHG under conditions previously shown to result in the generation of circular Ad5 genomes. Graham F.L., 1984, *EMBO J*. 3: 2917-2922; Ruben, M., Bacchetti, S. and Graham, F.L., 1983, *Nature* 301: 172-174. At 48 hours post-infection, DNA was extracted from the infected BRK cells and used to transform E. coli strain HMS 174 to ampicillin (Ap^{r}) and tetracycline resistance (Tet^{r}). From two experiments a total of 104 colonies were obtained. Small scale plasmid preparations were screened by HindII and BamHI/SmaI digestion and gel electrophoresis. The results of the restriction analysis revealed that the plasmids varied in the amount of the viral genome which they contained. This is believed to be due, at least in part, to the formation of a 206 bp palindrome when the inverted terminal repeats (ITR's) of the Ad5 genome are joined head to tail (the junction).

From the restriction analysis four candidate plasmids were selected that appeared to posses a complete AdBHG genome with intact junction regions. All four plasmids were found to be infectious in infectivity assays in which 293 cells were transfected with 5 or 10 µg of plasmid DNA (data not shown).

The ITR junctions in each of the infectious clones were sequenced and analyzed. The number of nucleotides missing from the mid point of the palindrome in each clone varied from as few as 4 bp (1 bp from the right ITR and 3 bp from the left) to as many as 19 bp (1 bp from the right ITR and 18 bp from the left). For further work we chose the clone missing 19 bp from the junction and called this pBHG9.

pBHG10 was generated by deleting the packaging signals in pBHG9. This was accomplished by partial BamHI digestion and religation (Fig. 1B). Screening for pBHG10 was facilitated by the fact that removal of the packaging signals also resulted in the elimination of the Tet^{r} gene.

pBHG10 contains Ad5 DNA sequences from bp 19 (left genomic end) to bp 188; bp 1339 to 28133; and bp 30818 to 35934 (right genomic end). The left and right termini of the Ad5 genomes are covalently joined. A segment of plasmid pBR322, representing nucleotides 375-1/4361-2064 of the pBR322 genome, which includes the pBR322 origin of replication and the pBR322 ampicillin resistance gene, is present between Ad5 bp 188 and 1339 to allow propagation of pBHG10 in host cells such as E. coli. A PacI restriction enzyme site, unique in this plasmid, is present between Ad5 bp 28133 and bp 30818 to permit insertion of foreign genes. Because the packaging signal is deleted, pBHG10 by itself does not yield infectious viral particles. Cotransfections of pBHG10 with helper plasmids containing the left end of Ad5 sequences, including the packaging signal, yields through recombination in the host cell infectious viral vectors with an efficiency comparable to that obtained using pJM17.¹
¹ Although pJM17 has been found useful for rescue of E1 mutations or substitutions into infectious virus, it has neither a wild type E3 region nor a useful E3 deletion. McGrory, W.J., Bautista, D.S. & Graham, F.L. (1988) *Virology* 163, 614-617 (unpublished and see below). Thus, pJM17 will be superseded by the pBHG series of plasmids for most Ad5 vector constructions.

### Example 2: Additional alterations to pBHG10: Insertion of wild type E3 sequences and substitution of the E3 region with an expanded deletion.

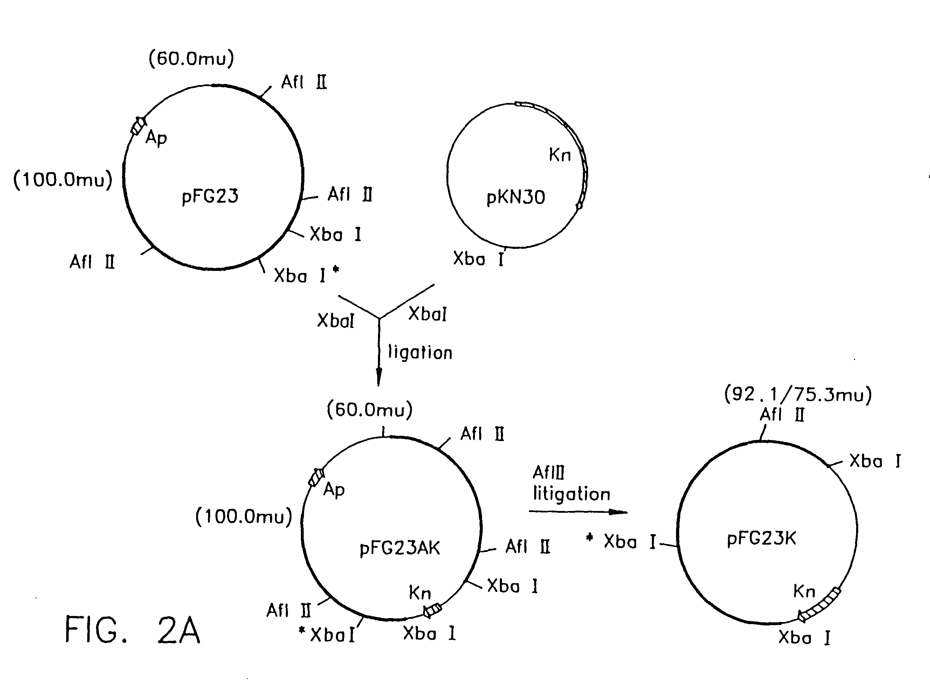

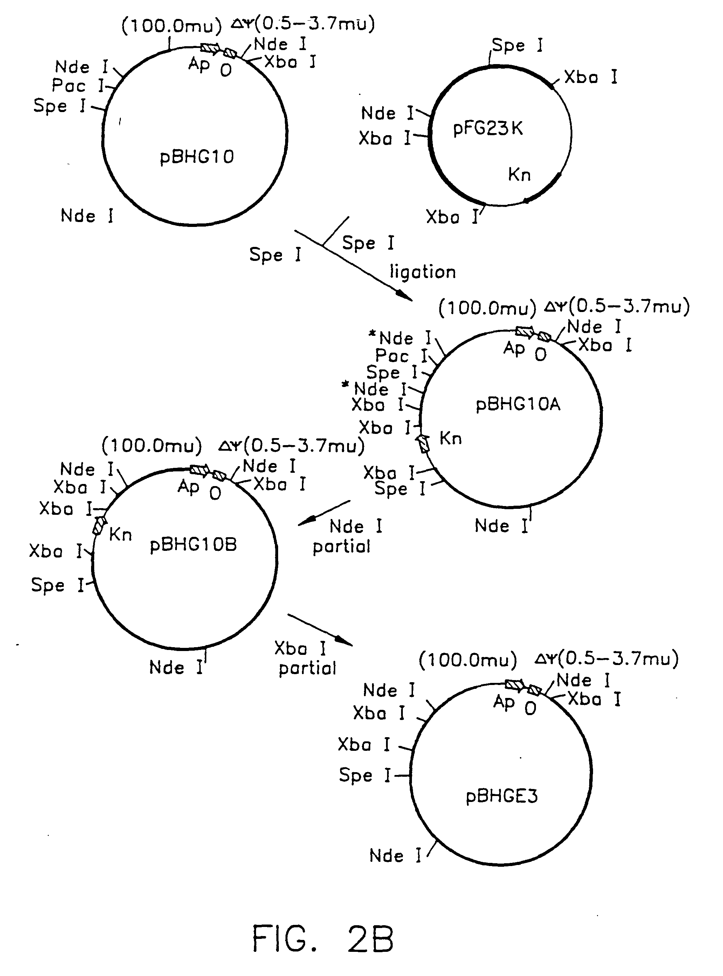

Since for some applications it may be desirable to generate Ad vectors with intact wild type Ad5 E3 sequences, we reintroduced wild type E3 sequences into pBHG10 (Fig. 2). The first step involved construction of a plasmid carrying E3 sequences flanking a kanamycin resistance (Kn^{r}) gene to simplify insertion into pBHG10. The Ap^{r} plasmid pFG23 (McKinnon, R.D., Bacchetti, S. and Graham, F.L., 1982, *Gene* 19: 33-42) was digested with XbaI, which cuts at position 28592 in Ad5 sequences (there is no cleavage at 30470 bp due to Dam methylation in the E. coli strain used) and ligated with XbaI-digested pKN30 (Lee, F., 1982, PH.D. Thesis, McMaster University, Hamilton, Ontario, Canada), generating pFG23AK (Ap^{r} and Kn^{r}) (Fig. 2A). To remove extraneous Ad5 sequences and the Ap^{r} gene, pFG23AK was digested with AflII and ligated, generating pFG23K.

The next step involved insertion of E3 sequences back into pBHG10 in the correct orientation (Fig. 2B). pBHG10 was digested with SpeI, which cuts only at 75.4 mu in Ad5 sequences, and ligated with pFG23K which had been linearized with SpeI, generating pBHGlOA which now contains the desired wild type E3 sequences in tandem with the previous E3 region containing the 2.69 kb deletion. To remove repeated sequences, pBHG10A was partially digested with NdeI and religated, generating pBHG1OB. In the final step the Kn^{r} segment was removed from pBHG1OB by partial XbaI digestion and religation, generating pBHGE3. Except for the presence of a wild type E3 region, pBHGE3 is identical to pBHG10, and is equally efficient for generation of Ad vectors with E1 substitution by cotransfection.

Our analysis of the sequences in the E3 region of Ad5 led us to believe it might be possible to expand the 2.69 kb deletion present in pBHG10 to 3.13 kb. By utilizing the technique of polymerase chain reaction (PCR) and following a strategy very similar to that described above for the construction of pBHGE3 (Fig. 2), we created a 3.13 kb E3 deletion and introduced it into pBHG10. The resulting plasmid pBHG11 is identical to pBHG10 except for an expanded E3 deletion which removes sequences from 27865 to 30995 bp. Like pBHG10, pBHG11 contains a unique PacI restriction enzyme site in place of the deleted E3 sequences to permit insertion of foreign genes.

### Example 3: Construction of E1 shuttle plasmids for use in cotransfections with pBHG vectors

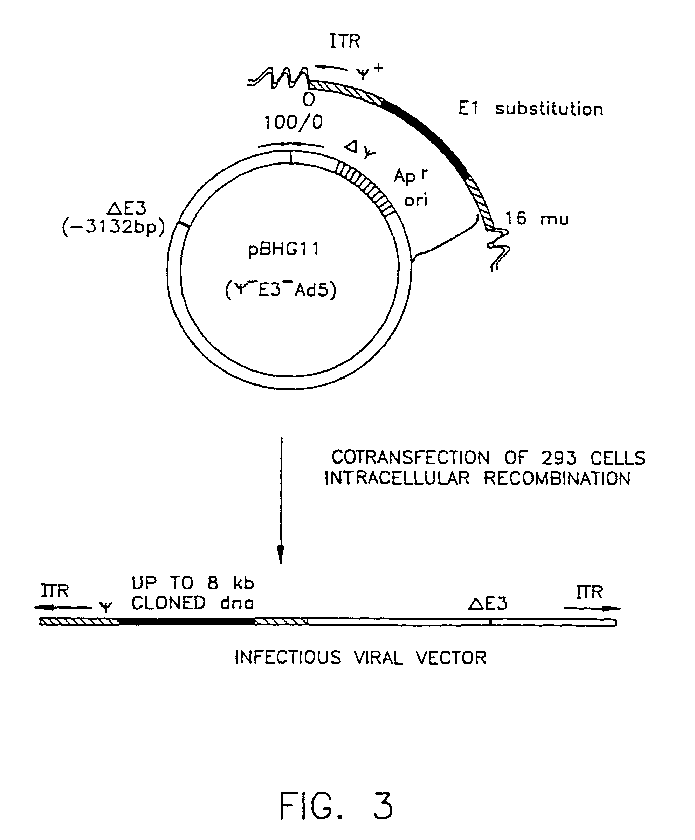

Plasmids pBHG10, pBHG11 and pBHGE3 were designed so that they would contain all the essential Ad5 sequences required to produce infectious virus upon transfection of 293 cells except for the packaging signal (194-358 bp) needed to encapsidate viral DNA into viral particles. To generate infectious viral vectors pBHG10, pBHG11, pBHGE3 or derivatives carrying an insert in E3 must be cotransfected into 293 cells with a second plasmid containing left end (E1) viral sequences including the packaging signal, as illustrated in Figure 3. To maximize the capacity of the BHG vector system we required a plasmid with the largest possible E1 deletion for co-transfections with the BHG plasmids.

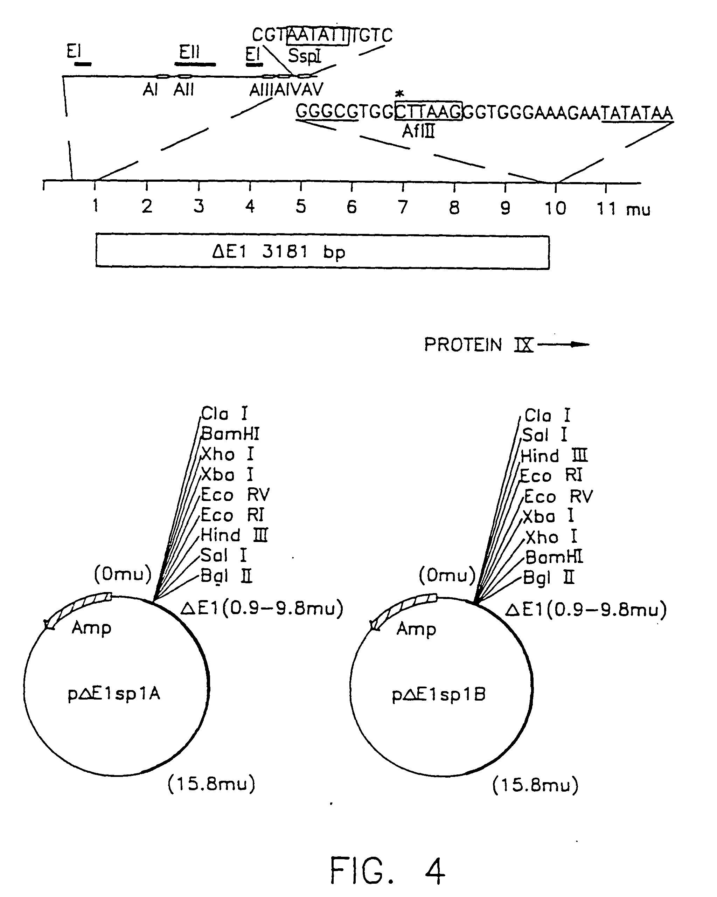

Our analysis of E1 sequences revealed that a deletion of approximately 3.2 kb could be created by removing the sequences between an Ssp I site at 339 bp and an Afl II site at 3533 bp (Fig. 4). This deletion does not interfere with the ITR (1-103 bp), the essential core packaging signal (194-358 bp) or coding sequences for protein IX but it does remove the sp1 binding site (3525-3530 bp) from the protein IX promoter. While this 3.2 kb E1 deletion does not interfere with the E1 enhancer region, it does remove the 3'-most, packaging element. The removal of this element has little or no effect on packaging.

Since the sp1 binding site is thought to be essential for protein IX expression, (Babiss, L.E. and Vales, L.D., 1991, *J. Virol*. 65: 598-605) it was reintroduced as a synthetic oligonucleotide which positioned the sp1 site 1 bp closer to the protein IX TATA box (Fig. 4).

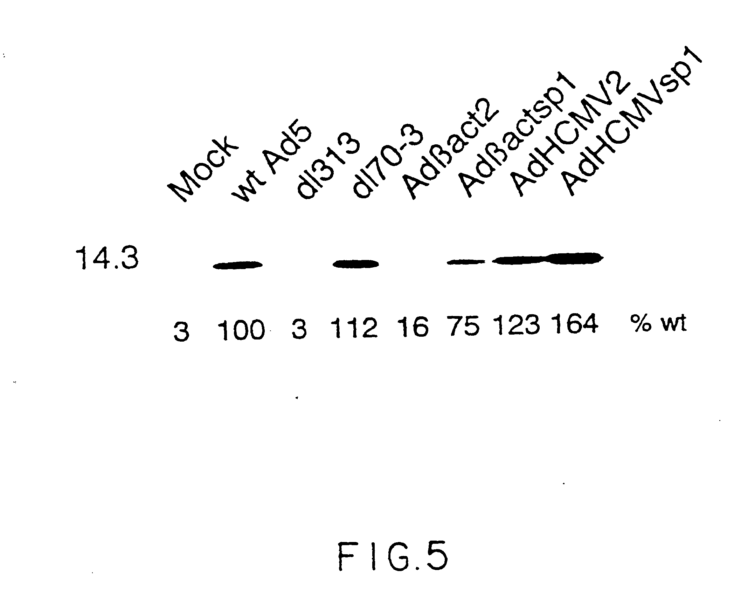

To assess the effect of the 3.2 kb E1 deletion and the reintroduction of the sp1 binding site, we examined protein IX expression by immunoprecipitation. 293 cells were infected at 10 PFU/cell with viruses containing either no deletion in E1 (wild type Ad5), a 2.3 kb deletion extending into the protein IX gene (d1313), the 3.2 kb deletion described above (d170-3), the 3.2 kb deletion containing the HCMV (AdHCMV2) or β-Actin (AdβAct2) promoters in the E1 antiparallel orientation or the 3.2 kb deletion containing the HCMV (AdHCMVsp1) or β-Actin (AdβActsp1) promoters with the reintroduced sp1 binding site. After labelling with [³⁵S]-methionine cell extracts were harvested, samples were immunoprecipitated with anti Ad2 protein IX antibodies and run on a 12% SDS PAGE gel. The results (Fig. 5) indicate that variable levels of protein IX were expressed depending on the sequences upstream from the protein IX gene but with the sp1 site present there was at most a 25% reduction compared to wild type Ad5.

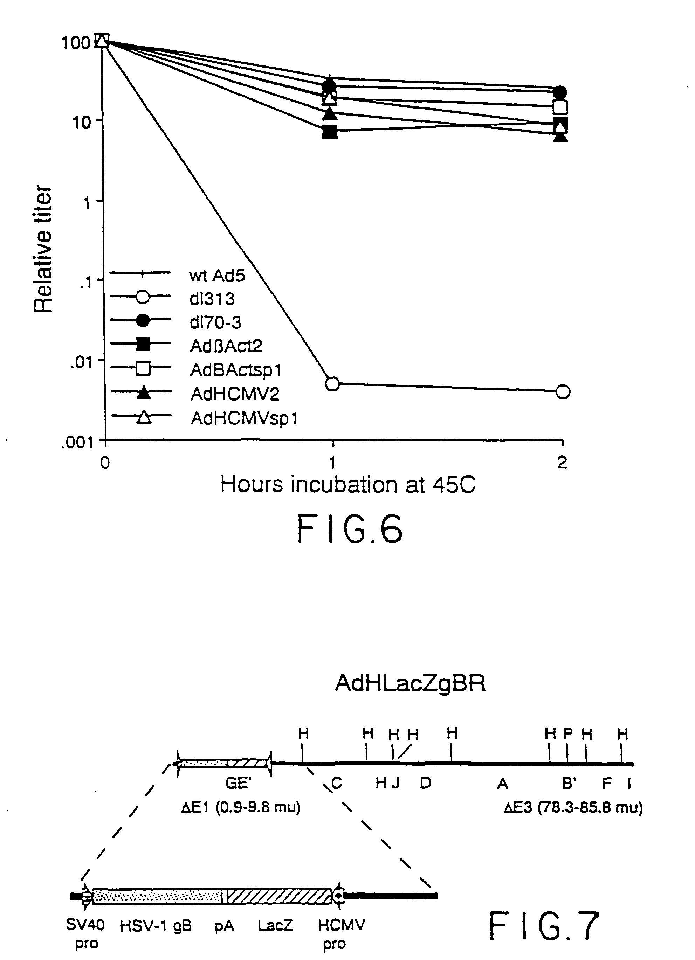

Because protein IX is known to affect the heat stability of virus particles we examined the heat stability of wild type Ad5 compared to d1313, d170-3, AdHCMV2, AdβAct2, AdHCMVsp1 and AdβActsp1. Stocks of these viruses were titered prior to and after incubation at 45°C for 1 and 2 hours. Of the six viral mutants tested only d1313 differed significantly in heat lability from wild type (Fig. 6). Even AdβAct2, which produces only 16% of wild type levels of protein IX (Fig. 5) was as resistant to heat inactivation as was wild type virus. This indicates that Protein IX is likely made in excess during viral infection. We have also found that viruses containing the 3.2 kb E1 deletion replicate in 293 cells to the same final titers as wild type Ad5 (data not shown).

With the verification that the growth characteristics and stability of viruses with the 3.2 kb E1 deletion were not affected it was decided to incorporate this deletion into plasmids pΔElsp1A and pΔElsp1B for use in cotransfections with the BHG plasmids (Fig. 4). These plasmids contain various restriction sites to facilitate the insertion of foreign genes.

The invention also includes a vector that includes a fragment or fragments of plasmid pBR322 which includes both an ampicillin resistance and the pBR322 origin of replication (which enables said vector to be replicated in cells wherein pBR322 is capable of being replicated), and an insert between early region 4 (E4) and the right inverted terminal repeat, and a deletion of E1 sequences from position 188 to or near the AflII sequence at position 3533, and cloning sites for the insertion of a foreign nucleic acid.

### Testing the efficiency and capacity of the pBHG vectors.

To assess the ability of the BHG plasmids to generate infectious viral vectors, cotransfections with various left end plasmids were performed and it was found that the efficiency of rescue was comparable to that obtained with pJM17 (data not shown).

The use of pBHGE3, pBHG10 or pBHG11 combined with the 3.2 kb deletion in E1 should permit rescue of inserts of approximately 5.2, 7.9 and 8.3 kb respectively into viral vectors. In order to test the capacity of the BHG system we constructed an insert of 7.8 kb consisting of the lacZ gene driven by the human cytomegalovirus immediate early promoter and the herpes simplex virus type 1 (HSV-1) gB gene driven by the SV40 promoter in the 3.2 kb E1 deletion (Fig. 7). Following cotransfection of 20-60 mm dishes of 293 cells, 10 with 5 µg each of pBHG10 and pHlacZgBR and the other half with 10 µg of each, one plaque was obtained. This was isolated, expanded, analyzed by restriction digest with HindIII and found to have the expected restriction pattern. The isolate designated AdHlacZgBR was found to express both lacZ and HSV-1 gB at levels comparable to that obtained with vectors containing single inserts of these genes (data not shown).

### Example 4: Additional shuttle plasmids

A shuttle vector, pABS.4, was used in the construction of a double recombinant containing lacZ in the E3 deletion and firefly luciferase in the E1 deletion. The construction of this vector further illustrates the use of the shuttle vectors as well as double recombinants.

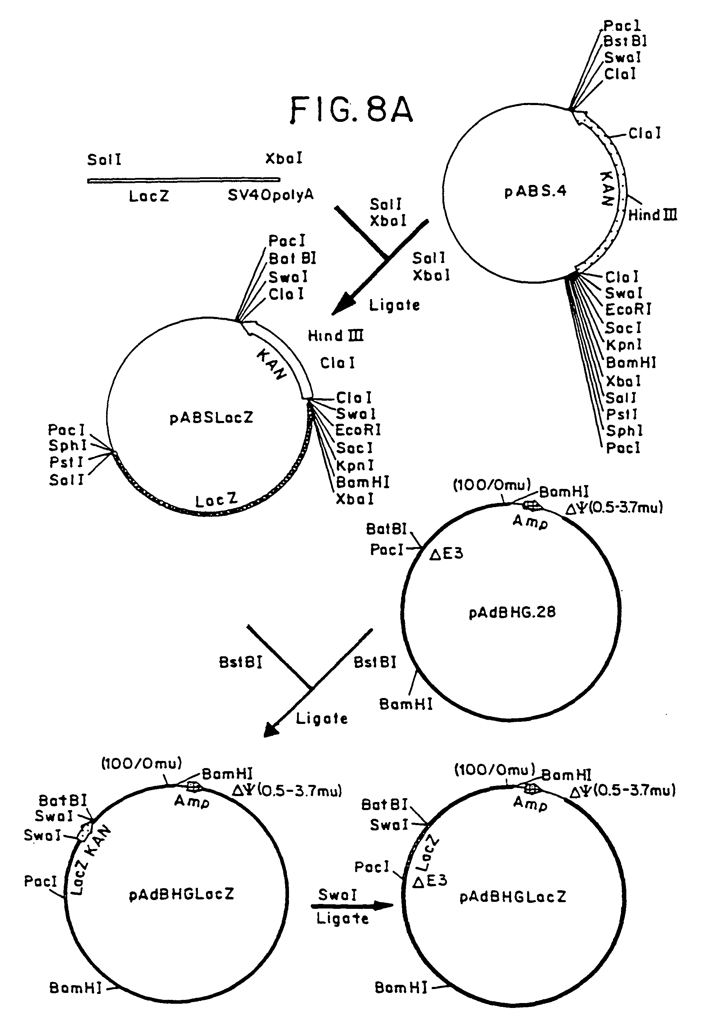

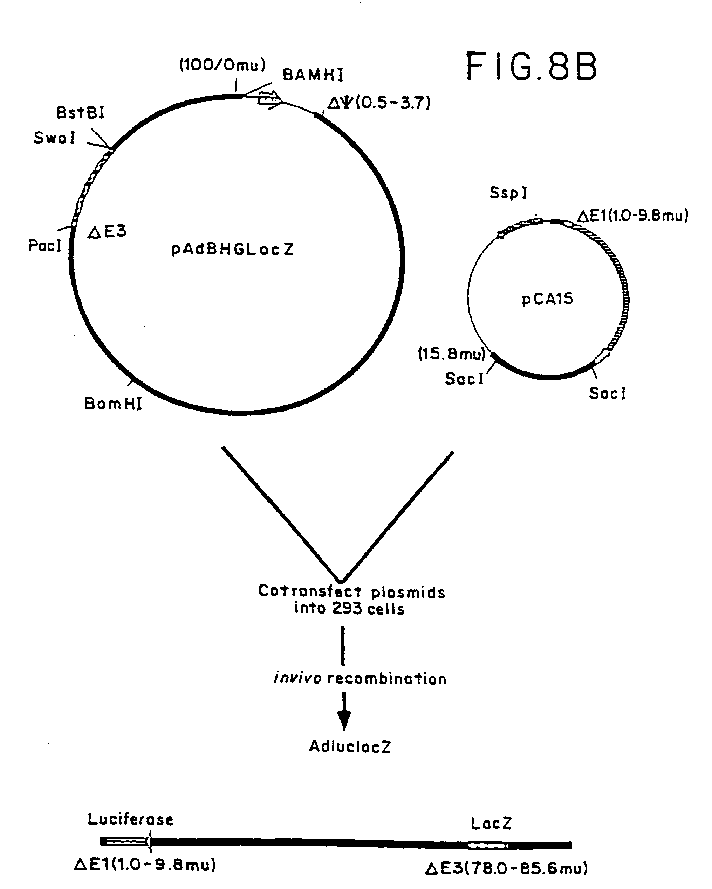

The strategy for the construction of this vector is presented in Fig. 8. First the lacZ gene with the SV40 poly A signal was inserted between the SalI and XbaI sites in the cloning region of pABS.4, generating pABSLacZ. Fig. 8A. In the next step pABSLacZ was digested with PacI and BstBI generating a fragment containing the LacZ gene and the Kanamycin resistance gene (Kan^{r}). This fragment was then inserted between the PacI and BstBI sites of pAdBHG.28, in the E3 parallel orientation, generating pAdBHGLacZK. Because double antibiotic selection was used, screening for the desired plasmid containing the lacZ insert was trivial. Finally pAdBHGLacZK was digested in SwaI to remove the Kan¹ gene generating pAdBHGLacZ. pAdBHGLacZ are grown and used in cotransfection with pCA15, a plasmid containing Ad5 left end sequences with firefly luciferase under the control of the human cytomegalovirus immediate early gene (HCMV) promoter in place of most of E1. Fig. 8B. pAdBHGLacZ can be used in cotransfections with virtually any E1 derived construct to make vectors with a variety of combinations of lacZ in E3 and foreign gene inserts or mutations in E1.

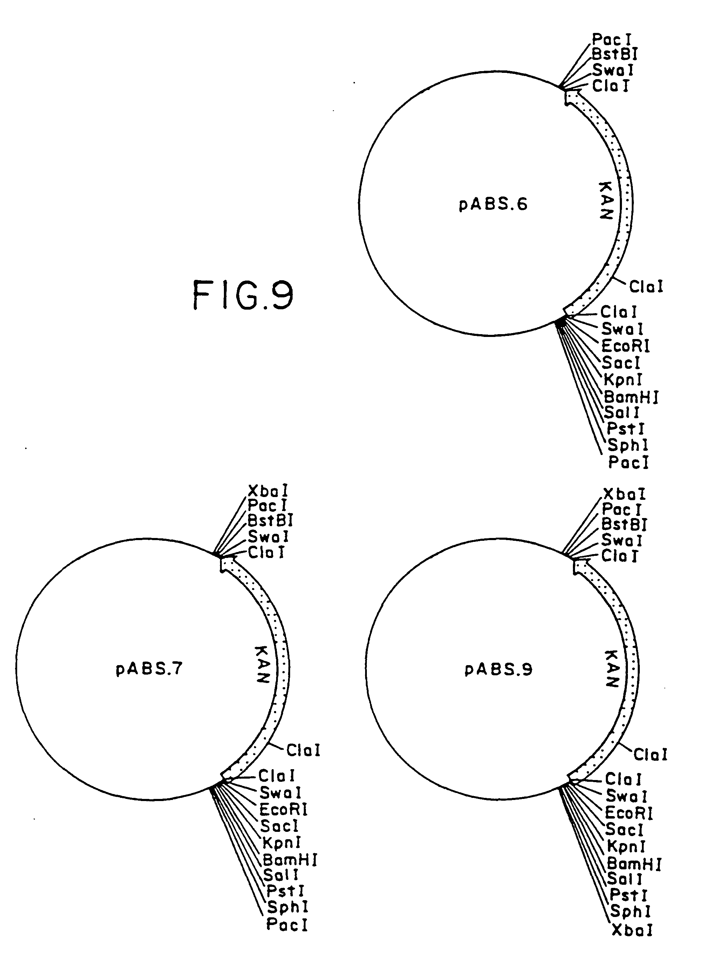

We developed shuttle vectors pABS.6, pABS.7 and pABS.9 to simplify the introduction of inserts into the E3 deletion in the pAdBHG plasmids. Fig. 9. They are used to facilitate transfer of foreign genes into the pAdBHG series of plasmids as follows: gene sequences are inserted into either pABS.7 or pABS.9 using cloning sites SphI, PstI, SalI, BamHI, KpnI, SacI, or EcoRI. The shuttle plasmid is then cut with one or two combinations of XbaI, PacI or BstBI and the Kan containing fragment is inserted into the Amp^{r} pAdBHG plasmid making use of Amp+Kan double resistance to select for bacterial transformants carrying the desired plasmid. Subsequently the Kan^{r} gene is removed by digestion with ClaI or SwaI and ligation. Finally the plasmid is "rescued" into infectious Ad viral vectors by cotransfection of 293 cells with an appropriate plasmid containing E1 sequences.

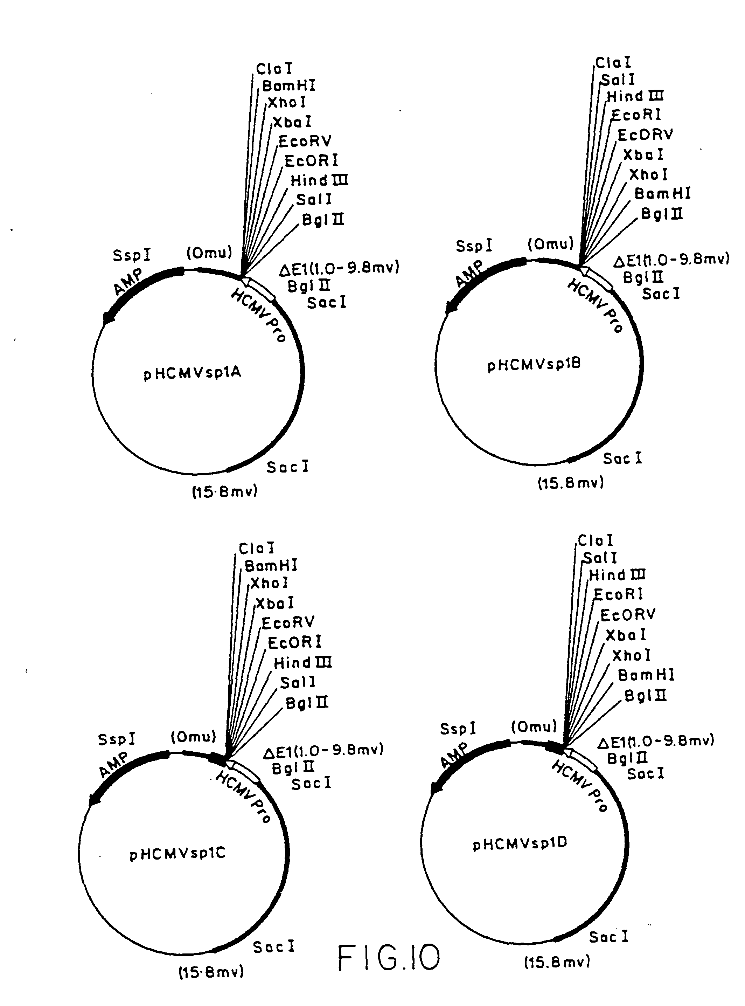

A number of shuttle plasmids have been constructed that can be used for cotransfections with vectors of the pGBH series. These are listed in Table I; see also Fig. 10. An E1 shuttle plasmid having a packaging signal inserted between early region 4 (E4) and the right inverted terminal repeat (ITR) is specifically part of the subject matter of the invention.

**Table 1.**

| **Additional E1 Shuttle Plasmids for Contransfection with pBHG Vectors** | | | |
|---|---|---|---|
| plasmid | regulatory sequences | net deletion | cloning sites |
| pXCJL1 | ----- | 2.88 kb | X-B-Xh-S-C |
| pXCJL2 | ----- | 2.88 kb | C-S-Xh-B-X |
| PΔE1sp1A | ----- | 3.19 kb | C-B-Xh-X-EV-E-H- |
| | | | S-Bg |
| pΔE1sp1B | ----- | 3.19 kb | C-S-H-E-EV-X-Xh- |
| | | | B-Bg |
| pHCMVsp1A | HCMV (L) | 2.81 kb | C-B-Xh-X-EV-E-H- |
| | | | S |
| pHCMVsp1B | HCMV (L) | 2.81 kb | C-S-H-E-EV-X-Xh- |
| | | | B |
| pHCMVsp1C | HCMV(L)/SV40pA | 2.66 kb | C-B-Xh-X-EV-E-H- |
| | | | S |
| pHCMVsp1D | HCMV(L)/SV40pA | 2.66 kb | C-S-H-E-EV-X-Xh- |
| | | | B |
| pCA3 | HCMV(L)/SV40pA | 2.66 kb | B-Xh-X-EV-E-H-S |
| pCA4 | HCMV(L)/SV40pA | 2.66 kb | S-H-E-EV-X-Xh-B |
| pCA13 | HCMV(R)/SV40pA | 2.66 kb | S-H-E-EV-X-Xh-B |
| pCA14 | HCMV(R)/SV40pA | 2.66 kb | B-Xh-X-EV-E-H-S |
| pBActsp1A | βActin(L) | 1.74 kb | C-B-X-EV-E-H-S |
| pBActsp1B | βActin(L) | 1.74 kb | C-S-H-E-EV-X-B |
| pCA1 | βActin(L)/SV40pA | 1.58 kb | S-H-E-EV-X-B |
| pCA2 | βActin(L)/SV40pA | 1.58 kb | B-X-EV-E-H-S |
| pMLPsp1A | MLP(R) | 2.23 kb | B-X-EV-E-H-S-Bg |
| X: XbaI, B: BamHI, Xh: XhOI, S: SalI, C: ClaI, EV: EcoRV, E: EcoRI, H: HindIII, Bg: BglII | | | |

The above described experimental methods are not intended to be limiting. Those skilled in the art will appreciate that a variety of methods may be used to introduce vectors into the cells of target tissues (for example, liver tumors might be treated by infusing the affected liver via the portal vein with vectors of the kind described and/or claimed herein and in the parent applications). In addition, the invention contemplates the use of vectors containing foreign nucleic acids that encode molecules that may be useful to treat diseases, such as antisense RNA, tissue growth factors such as GM-CSF, molecules that trigger differentiation, molecules that induce apoptosis, etc. Finally, a person skilled in the art will appreciate that the methods of this invention can be used to treat animals other than mice and humans.

## Claims

1. A vector system, comprising:
(a) a first plasmid having a modified adenovirus genome having
(i) a modification within the early region 1 (E1) of said genome that eliminates the packaging signal in said genome;
and
(ii) a fragment or fragments of a bacterial plasmid which encode an antibiotic resistance and include the plasmid's origin of replication and other sequences necessary to allow the vector to be replicated in cells wherein the bacterial plasmid is capable of being replicated; and
(b) a second plasmid that serves as an E1 shuttle plasmid, comprising:
(i) a fragment or fragments of a bacterial plasmid which encode an antibiotic resistance and include the plasmid's origin of replication and other sequences necessary to allow the vector to be replicated in cells wherein the bacterial plasmid is capable of being replicated,
and
(ii) a sequence derived from an Ad E1 region,
(c) wherein said first and second plasmids are capable of recombining to produce a recombinant modified adenovirus genome that is capable of being packaged into viral particles.

2. The vector system of claim 1 wherein the second plasmid further comprises a foreign nucleic acid spliced within said sequence derived from an Ad E1 region and wherein said recombinant modified adenovirus genome produced by recombination of said first and second plasmids contains said foreign nucleic acid.

3. The vector system of claim 1, wherein the modified adenovirus genome of the first plasmid is derived from the genome of adenovirus 5 (Ad5).

4. The vector system of claim 1, wherein said modification in E1 is a deletion that comprises the E1A region.

5. The vector system of claim 1, wherein the modification in E1 is a deletion that spans nucleotides 188 to 1339.

6. The vector system of claim 1, wherein the modification in E1 is a deletion that spans nucleotide 188 and the Af1II site at position 3533.

7. The vector system of claim 1 wherein an sp1 site at position 3525 that was deleted from said first plasmid is reintroduced into the second plasmid by inserting a synthetic oligonucleotide that includes an sp1 site.

8. The vector system of claim 1, wherein said first plasmid further comprises a deletion within early region 3 (E3), wherein said E3 deletion does not inhibit the expression of sequences necessary for viral replication, packaging, viability, or infectivity.

9. The vector system of claim 8 wherein, wherein the E3 deletion comprises positions 27865-30995 of the Ad5 genome.

10. The vector system of claim 1, wherein said first plasmid additionally comprises a fragment or fragments of bacterial plasmid pBR322 which encodes an ampicillin resistance, and also a pBR322 origin of replication which enables said first plasmid to be replicated in cells wherein pBR322 is capable of being replicated.

11. The vector system of claim 1 wherein said plasmid fragment or fragments according to claim 1(a)(ii) are inserted at a location selected from the group of locations that consists of: a location between early region 4 (E4) and the right inverted terminal repeat (ITR); a location within early region 4; and a location wherein sequences spanning E4 are deleted and substituted with said plasmid fragment or fragments.

12. The vector system of claim 1 wherein said first plasmid further comprises cloning sites for the insertion of a nucleic acid sequence.

13. The vector system of claim 1, said first plasmid comprising a fragment or fragments of bacterial plasmid pBR322 which encodes an ampicillin resistance and also a pBR322 origin of replication which enables said vector to be replicated in cells wherein pBR322 is capable of being replicated, wherein said vector is further modified to contain an insert between early region 4 (E4) and the right inverted terminal repeat, which vector further has a deletion of E1 sequences from position 188 to or near the Af1II sequence at position 3533, wherein said vector contains cloning sites for the insertion of a foreign nucleic acid.

14. A plasmid comprising adenovirus Ad5 DNA sequences from bp 19 (left genomic terminus) to bp188; bp 1339 to 28133; and bp 30818 to bp 35934 (right genomic terminus) wherein the left and right genomic termini of the Ad5 sequences are covalently joined, and further comprising a fragment of plasmid pBR322 consisting of nucleotides 375-1/4361-2064 inserted between Ad 5 bp 188 and bp 1339 and a *PacI* restriction site positioned between Ad5 bp 28133 and bp 30818.

15. A plasmid comprising adenovirus Ad5 DNA sequences from bp 19 (left genomic terminus) to bp188; bp 1339 to bp 35934 (right genomic terminus) wherein the left and right genomic termini of the Ad5 sequences are covalently joined, and further comprising a fragment of plasmid pBR322 consisting of nucleotides 375-1/4361-2064 inserted between Ad5 bp 188 and bp 1339.

16. A plasmid comprising adenovirus Ad5 DNA sequences from bp 19 (left genomic terminus) to bp188; bp 1339 to 27865; and bp 30995 to bp 35934 (right genomic terminus) wherein the left and right genomic termini of the Ad5 sequences are covalently joined, and further comprising a fragment of plasmid pBR322 consisting of nucleotides 375-1/4361-2064 inserted between Ad 5 bp 188 and bp 1339 and a *PacI* restriction site positioned between Ad5 bp 27865 and bp 30995.

17. The vector system of claim 1, wherein the second plasmid is an E1 shuttle plasmid that comprises said sequence derived from the Ad5 E1 region, including a packaging signal.

18. The vector system of claim 17, wherein a foreign nucleic acid that comprises an open reading frame is inserted into said E1 shuttle plasmid.

19. The vector system of claim 18, where sequences that regulate the expression of said open reading frame are further inserted into said E1 shuttle plasmid.

20. The vector system of claim 17, wherein said E1 shuttle plasmid comprises a deletion for the sequence derived from the Ad5 E1 region for the fragment of approximately 3.19 kb between an SspI site at 339 bp and a AflII site at 3533 bp, which plasmid further comprises a synthetic sp1 binding site and cloning sites for the insertion of a foreign nucleic acid within the E1 deletion.

21. The vector system of claim 17, wherein said E1 shuttle plasmid comprises a deletion in the sequence derived from the Ad5 E1 region, and further comprises an HCMV promoter positioned within the E1 deletion and cloning sites for the insertion of a foreign nucleic acid positioned adjacent to said HCMV promoter within the E1 deletion.

22. The vector system of claim 17, wherein said E1 shuttle plasmid comprises a deletion in the sequence derived from the Ad5 E1 region and which further comprises an HCMV promoter and an SV40 polyadenylation signal both positioned within the E1 deletion and cloning sites for the insertion of a foreign nucleic acid positioned between the HCMV promoter and the SV40 polyadenylation signal.

23. The vector system of claim 17, wherein said E1 shuttle plasmid comprises a deletion of approximately 2.88 kb in the sequence derived from the Ad5 E1 region and which further comprises cloning sites for the insertion of a foreign nucleic acid positioned within the E1 deletion.

24. The vector system of claim 17, wherein said E1 shuttle plasmid comprises a deletion in the sequence derived from the Ad5 E1 region and which further comprises a β actin promoter and an SV40 polyadenylation signal both positioned within the E1 deletion and cloning sites for the insertion of a foreign nucleic acid positioned between the β actin promoter and the SV40 polyadenylation signal.

25. The vector system of claim 17, wherein said E1 shuttle plasmid comprises a packaging signal inserted between early region 4 (E4) and the right inverted terminal repeat (ITR).

26. An in vitro method for introducing and expressing a foreign nucleic acid in a host cell, comprising:
(a) introducing
(i) a first plasmid comprising a modified adenovirus genome which comprises a modification within the early region 1 (E1) of said genome that eliminates the packaging signal in said genome;
and
(ii) an E1 shuttle plasmid that contains a sequence derived from an Ad E1 region, including an encapsidation signal and an inserted nucleic acid sequence, into a host cell that expresses functions encoded by the E1 sequence that have been deleted from the first plasmid;
and
(b) isolating a recombinant viral genome wherein the first plasmid has recombined with the E1 shuttle plasmid to yield a recombinant modified viral genome containing the encapsidation signal and the inserted nucleic acid sequence of the E1 shuttle plasmid and elements of the first plasmid excluding the modification within E1,
(c) introducing said recombinant modified viral genome into a host cell, and
(d) expressing the coding sequences contained in said modified recombinant viral genome.

27. A method according to claim 26, wherein said first plasmid further comprises a fragment or fragments of a bacterial plasmid which encode an antibiotic resistance and include the plasmid's origin of replication and other sequences necessary to allow the vector to be replicated in cells wherein the plasmid is capable of being replicated.

28. A method according to claims 26 or claim 27, wherein into said E1 shuttle plasmid is further inserted a nucleic acid sequence that comprises an open reading frame.

29. A method according to claim 28 wherein into said E1 shuttle plasmid are further inserted sequences that regulate the expression of said open reading frame.

30. A method according to any one of claims 26 to 29, wherein said recombinant viral genome is packaged into a recombinant viral particle that is capable of infecting cells which can be infected by the adenovirus from which the modified adenovirus genome was originally derived.

31. A method according to any one of claims 26 to 30, wherein said modified adenovirus genome was originally derived from adenovirus 5.

32. A method according to any one of claims 26 to 31, wherein said vector having an E1 deletion is introduced into said host cells together with an adenoviral particle having an intact E1 region, wherein said adenoviral particle having an intact E1 region is capable of complementing the replication of said vector having an E1 deletion.

## Patentansprüche

1. Vektorsystem, das folgendes aufweist:
(a) ein erstes Plasmid, das ein modifiziertes Adenovirus-Genom hat, das folgendes hat:
(i) eine Modifikation innerhalb des frühen Bereichs 1 (E1) des Genoms, die das Packungssignal in dem Genom eliminiert;
und
(ii) ein Fragment oder Fragmente eines bakteriellen Plasmids, die für eine Antibiotikaresistenz codieren und den Replikationsursprung des Plasmids und andere Sequenzen aufweisen, die erforderlich sind, um zuzulassen, daß der Vektor in Zellen repliziert wird, in denen das baktierielle Plasmid repliziert werden kann;
und
(b) ein zweites Plasmid, das als ein E1-Shuttle-Plasmid dient und folgendes aufweist:
(i) ein Fragment oder Fragmente eines bakteriellen Plasmids, die für eine Antibiotikaresistenz codieren und den Replikationsursprung des Plasmids und andere Sequenzen aufweisen, die erforderlich sind, um zuzulassen, daß der Vektor in Zellen repliziert wird, in denen das bakterielle Plasmid repliziert werden kann,
und
(ii) eine von einem Ad-E1-Bereich abgeleitete Sequenz,
(c) wobei das erste und das zweite Plasmid rekombinierbar ist, um ein rekombinantes modifiziertes Adenovirus-Genom zu produzieren, das imstande ist, in virale Partikel gepackt zu werden.

2. Vektorsystem nach Anspruch 1, wobei das zweite Plasmid ferner eine Fremd-Nucleinsäure aufweist, die innerhalb der von einem Ad-E1-Bereich abgeleiteten Sequenz gespleißt ist, und wobei das durch Rekomination des ersten und des zweiten Plasmids prozudierte rekombinante modifizierte Adenovirus-Genom diese Fremd-Nucleinsäure enthält.

3. Vektorsystem nach Anspruch 1, wobei das modifizierte Adenovirus-Genom des ersten Plasmids von dem Genom von Adenovirus 5 (Ad5) abgeleitet ist.

4. Vektorsystem nach Anspruch 1, wobei die Modifikation in E1 eine Deletion ist, die den E1A-Bereich umfaßt.

5. Vektorsystem nach Anspruch 1, wobei die Modifikation in E1 eine Deletion ist, welche die Nucleotide 188 bis 1339 umspannt.

6. Vektorsystem nach Anspruch 1, wobei die Modifikation in E1 eine Deletion ist, die das Nucleotid 188 und die Af1II-Stelle an Position 3533 umspannt.

7. Vektorsystem nach Anspruch 1, wobei eine sp1-Stelle an Position 3525, die von dem ersten Plasmid deletiert wurde, in das zweite Plasmid wieder eingeführt wird, indem ein synthetisches Oligonucleotid eingefügt wird, das eine sp1-Stelle aufweist.

8. Vektorsystem nach Anspruch 1, wobei das erste Plasmid ferner eine Deletion innerhalb des frühen Bereichs 3 (E3) aufweist, wobei die E3-Deletion die Exprimierung von Sequenzen nicht hemmt, die erforderlich sind für virale Replikation, Packen, Lebensfähigkeit oder Infektionsvermögen.

9. Vektorsystem nach Anspruch 8, wobei die E3-Deletion Positionen 27865-30995 des Ad5-Genoms umfaßt.

10. Vektorsystem nach Anspruch 1, wobei das erste Plasmid zusätzlich aufweist: ein Fragment oder Fragmente von bakteriellem Plasmid pBR322, das für eine Ampicillinresistenz codiert, und ferner einen pBR322-Replikationsursprung, der ermöglicht, daß das erste Plasmid in Zellen repliziert wird, in denen pBR322 repliziert werden kann.

11. Vektorsystem nach Anspruch 1, wobei das Plasmidfragment oder die Plasmidfragmente nach Anspruch 1 (a) (ii) an einer Stelle eingefügt sind, die aus der Gruppe von Stellen ausgewählt ist, die besteht aus: einer Stelle zwischen dem frühen Bereich 4 (E4) und der rechten invertierten terminalen Sequenzwiederholung (ITR); einer Stelle innerhalb des frühen Bereichs 4; und einer Stelle, an der E4 umspannende Sequenzen deletiert und mit dem Plasmidfragment oder den Plasmidfragmenten substituiert sind.

12. Vektorsystem nach Anspruch 1, wobei das erste Plasmid ferner Klonierungsstellen für die Insertion einer Nucleinsäuresequenz aufweist.

13. Vektorsystem nach Anspruch 1, wobei das erste Plasmid aufweist: ein Fragment oder Fragment von bakteriellem Plasmid pBR322, das für eine Ampicillinresistenz codiert, und ferner einen pBR322-Replikationsursprung, der ermöglicht, daß der Vektor in Zellen repliziert wird, in denen pBR322 repliziert werden kann, wobei der Vektor ferner modifiziert ist, um ein Insert zwischen dem frühen Bereich 4 (E4) und der rechten invertierten terminalen Sequenzwiederholung zu enthalten, wobei der Vektor ferner eine Deletion von E1-Sequenzen von Position 188 bis zu oder in die Nähe der Af1II-Sequenz an Position 3533 hat, wobei der Vektor Klonierungsstellen für die Insertion einer Fremd-Nucleinsäure enthält.

14. Plasmid, das aufweist: Adenovirus-Ad5-DNA-Sequenzen von bp 19 (linker Genom-Terminus) bis bp188; bp 1339 bis 28133; und bp 30818 bis bp 35934 (rechter Genom-Terminus), wobei die linken und rechten Genom-Termini der Ad5-Sequenzen kovalent gebunden sind, und ferner aufweist: ein Fragment von Plasmid pBR322, das aus den Nucleotiden 375-1/4361-2064 besteht, die zwischen Ad 5 bp 188 und bp 1339 eingefügt sind, und eine *PacI*-Restriktions-Schnittstelle, die zwischen Ad5 bp 28133 und bp 30818 positioniert ist.

15. Plasmid, das aufweist: Adenovirus-Ad5-DNA-Sequenzen von bp 19 (linker Genom-Terminus) bis bp188; bp 1339 bis bp 35934 (rechter Genom-Terminus), wobei die linken und rechten Genom-Termini der Ad5-Sequenzen kovalent gebunden sind, und ferner aufweist: ein Fragment von Plasmid pBR322, das aus den Nucleotiden 375-1/4361-2064 besteht, die zwischen Ad 5 bp 188 und bp 1339 eingefügt sind.

16. Plasmid, das aufweist: Adenovirus-Ad5-DNA-Sequenzen von bp 19 (linker Genom-Terminus) bis bp188; bp 1339 bis 27865; und bp 30995 bis bp 35934 (rechter Genom-Terminus), wobei die linken und rechten Genom-Termini der Ad5-Sequenzen kovalent gebunden sind, und ferner aufweist: ein Fragment von Plasmid pBR322, das aus den Nucleotiden 375-1/4361-2064 besteht, die zwischen Ad 5 bp 188 und bp 1339 eingefügt sind, und eine *PacI*-Restriktions-Schnittstelle, die zwischen Ad5 bp 27865 und bp 30995 positioniert ist.

17. Vektorsystem nach Anspruch 1, wobei das zweite Plasmid ein E1-Shuttle-Plasmid ist, das die genannte von dem Ad5-E1-Bereich abgeleitete Sequenz aufweist, einschließlich eines Packungssignals.

18. Vektorsystem nach Anspruch 17, wobei eine Fremd-Nucleinsäure, die ein offenes Leseraster aufweist, in das E1-Shuttle-Plasmid eingefügt ist.

19. Vektorsystem nach Anspruch 18, wobei ferner Sequenzen, welche die Exprimierung des offenen Leserasters regulieren, in das E1-Shuttle-Plasmid eingefügt sind.

20. Vektorsystem nach Anspruch 17, wobei das E1-Shuttle-Plasmid eine Deletion für die von dem Ad5-E1-Bereich abgeleitete Sequenz für das Fragment von ungefähr 3,19 kb zwischen einer SspI-Stelle bei 339 bp und einer Af1II-Stelle bei 3533 bp aufweist, wobei das Plasmid ferner eine synthetische sp 1-Bindungsstelle und Klonierungsstellen für die Insertion einer Fremd-Nucleinsäure innerhalb der E1-Deletion aufweist.

21. Vektorsystem nach Anspruch 17, wobei das E1-Shuttle-Plasmid eine Deletion in der von dem Ad5-E1-Bereich abgeleiteten Sequenz aufweist, und ferner aufweist: einen HCMV-Promotor, der innerhalb der E1-Deletion positioniert ist, und Klonierungsstellen für die Insertion einer Fremd-Nucleinsäure, die dem HCMV-Promoter innerhalb der E1-Deletion benachbart positioniert sind.

22. Vektorsystem nach Anspruch 17, wobei das E1-Shuttle-Plasmid eine Deletion in der von dem Ad5-E1-Bereich abgeleiteten Sequenz aufweist, und ferner aufweist: einen HCMV-Promotor und ein SV40-Polyadenylierungssignal, die beide innerhalb der E1-Deletion positioniert sind, und Klonierungsstellen für die Insertion einer Fremd-Nucleinsäure, die zwischen dem HCMV-Promotor und dem SV40-Polyadenylierungssignal positioniert sind.

23. Vektorsystem nach Anspruch 17, wobei das E1-Shuttle-Plasmid eine Deletion von ungefähr 2,88 kb in der von dem Ad5-E1-Bereich abgeleiteten Sequenz aufweist, und ferner Klonierungsstellen für die Insertion einer Fremd-Nucleinsäure aufweist, die innerhalb der E1-Deletion positioniert sind.

24. Vektorsystem nach Anspruch 17, wobei das E1-Shuttle-Plasmid eine Deletion in der von dem Ad5-E1-Bereich abgeleiteten Sequenz aufweist, und ferner aufweist: einen β-Actin-Promotor und ein SV40-Polyadenylierungssignal, die beide innerhalb der E1-Deletion positioniert sind, und Klonierungsstellen für die Insertion einer Fremd-Nucleinsäure, die zwischen dem β-Actin-Promotor und dem SV40-Polyadenylierungssignal positioniert sind.

25. Vektorsystem nach Anspruch 17, wobei das E1-Shuttle-Plasmid ein Packungssignal aufweist, das zwischen den frühen Bereich 4 (E4) und die rechte invertierte terminale Sequenzwiederholung (ITR) eingefügt ist.

26. *In vitro* Verfahren zum Einführen und Exprimieren einer Fremd-Nucleinsäure in eine Wirtszelle, wobei das Verfahren die folgenden Schritte aufweist:
(a) Einführen
(i) eines ersten Plasmids, das ein modifiziertes Adenovirus-Genom aufweist, das eine Modifikation innerhalb des frühen Bereichs 1 (E1) des Genoms aufweist, die das Packungssignal in dem Genom eliminiert;
und
(ii) eines E1-Shuttle-Plasmids, das eine von einem Ad-E1-Bereich abgeleitete Sequenz enthält, einschließlich eines Enkapsidationssignals und einer eingefügten Nucleinsäuresequenz, in eine Wirtszelle, welche durch die E1-Sequenz codierte Funktionen exprimiert, die von dem ersten Plasmid deletiert worden sind;
und
(b) Isolieren eines rekombinanten viralen Genoms, in dem das erste Plasmid mit dem E1-Shuttle-Plasmid rekombiniert wurde, um ein rekombinantes modifiziertes virales Genom zu ergeben, welches das Enkapsidationssignal und die eingefügte Nucleinsäuresequenz des E1-Shuttle-Plasmids und Elemente des ersten Plasmids unter Ausschluß der Modifikation innerhalb von E1 enthält,
(c) Einführen des rekombinanten modifizierten viralen Genoms in eine Wirtszelle, und
(d) Exprimieren der codierenden Sequenzen, die in dem modifizierten rekombinanten viralen Genom enthalten sind.

27. Verfahren nach Anspruch 26, wobei das erste Plasmid ferner ein Fragment oder Fragmente eines bakteriellen Plasmids aufweist, die für eine Antibiotikaresistenz codieren und den Replikationsursprung des Plasmids und andere Sequenzen aufweisen, die erforderlich sind, um zuzulassen, daß der Vektor in Zellen repliziert wird, in denen das Plasmid repliziert werden kann.

28. Verfahren nach Anspruch 26 oder Anspruch 27, wobei in das E1-Shuttle-Plasmid ferner eine Nucleinsäuresequenz eingefügt wird, die ein offenes Leseraster aufweist.

29. Verfahren nach Anspruch 28, wobei in das E1-Shuttle-Plasmid ferner Sequenzen eingefügt werden, welche die Exprimierung des offenen Leserasters regulieren.

30. Verfahren nach einem der Ansprüche 26 bis 29, wobei das rekombinante virale Genom in ein rekombinantes virales Partikel gepackt wird, das imstande ist, Zellen zu infizieren, die durch das Adenovirus, von dem das modifizierte Adenovirus-Genom ursprünglich abgeleitet wurde, infiziert werden können.

31. Verfahren nach einem der Ansprüche 26 bis 30, wobei das modifizierte Adenovirus-Genom ursprünglich von Adenovirus 5 abgeleitet wurde.

32. Verfahren nach einem der Ansprüche 26 bis 31, wobei der Vektor, der eine E1-Deletion hat, gemeinsam mit einem adenoviralen Partikel, das einen intakten E1-Bereich hat, in die Wirtszellen eingeführt wird, wobei das adenovirale Partikel einen intakten E1-Bereich hat, der imstande ist, die Replikation des Vektors, der eine E1-Deletion hat, zu komplementieren.

## Revendications

1. Système de vecteurs, comprenant :
(a) un premier plasmide présentant un génome d'adénovirus modifié présentant :
(i) une modification au sein de la région précoce 1 (E1) dudit génome qui élimine le signal d'emballage dans ledit génome ; et
(ii) un fragment ou des fragments d'un plasmide bactérien qui code une résistance antibiotique et comprend l'origine de réplication du plasmide et autres séquences nécessaires pour permettre au vecteur d'être répliqué dans des cellules dans lesquelles le plasmide bactérien est capable d'être répliqué ;
et
(b) un deuxième plasmide servant de plasmide navette E1, comprenant :
(i) un fragment ou des fragments d'un plasmide bactérien qui code une résistance antibiotique et comprend l'origine de réplication du plasmide et autres séquences nécessaires pour permettre au vecteur d'être répliqué dans des cellules dans lesquelles le plasmide bactérien est capable d'être répliqué ; et
(ii) une séquence dérivée d'une région E1 d'Ad ;
(c) dans lequel lesdits premier et deuxième plasmides sont capables de se recombiner pour produire un génome d'adénovirus modifié recombinant capable d'être emballé dans des particules virales.

2. Système de vecteurs selon la revendication 1, dans lequel le deuxième plasmide comprend en outre un acide nucléique étranger épissé au sein de ladite séquence dérivée d'une région E1 d'Ad et dans lequel ledit génome d'adénovirus modifié recombinant produit par recombinaison desdits premier et deuxième plasmides renferme ledit acide nucléique étranger.

3. Système de vecteurs selon la revendication 1, dans lequel le génome d'adénovirus modifié du premier plasmide est dérivé du génome de l'adénovirus 5 (Ad5).

4. Système de vecteurs selon la revendication 1, dans lequel ladite modification en E1 est une délétion qui comprend la région E1A.

5. Système de vecteurs selon la revendication 1, dans lequel la modification en E1 est une délétion qui s'étend aux nucléotides 188 à 1339.

6. Système de vecteurs selon la revendication 1, dans lequel la modification en E1 est une délétion qui s'étend au nucléotide 188 et au site Af1II au niveau de la position 3533.

7. Système de vecteurs selon la revendication 1, dans lequel un site sp1 au niveau d'une position 3525 qui a été délété dudit premier plasmide est réintroduit dans le deuxième plasmide en insérant un oligonucléotide synthétique qui comprend un site sp1.

8. Système de vecteurs selon la revendication 1, dans lequel ledit premier plasmide comprend en outre une délétion au sein de la région précoce 3 (E3), dans lequel ladite délétion de E3 n'inhibe pas l'expression de séquences nécessaires pour la réplication, l'emballage, la visibilité ou le pouvoir infectant viral.

9. Système de vecteurs selon la revendication 8, dans lequel la délétion d'E3 comprend les positions 27865 - 30995 du génome d'Ad5.

10. Système de vecteurs selon la revendication 1, dans lequel ledit premier plasmide comprend en outre un fragment ou des fragments de plasmide bactérien pBR322 qui code une résistance à l'ampicilline et également une origine de réplication de pBR322 qui permet audit premier plasmide d'être répliqué dans des cellules dans lesquelles pBR322 est capable d'être répliqué.

11. Système de vecteurs selon la revendication 1, dans lequel ledit fragment ou lesdits fragments de plasmide selon la revendication 1 (a) (ii) sont insérés au niveau d'un emplacement sélectionné dans le groupe d'emplacements qui consiste en : un emplacement entre la région précoce 4 (E4) et les séquences répétées inversées terminales droites ; un emplacement au sein de la région précoce 4 ; et un emplacement dans lequel les séquences s'étendant à E4 sont délétées et substituées par ledit fragment ou lesdits fragments plasmide.

12. Système de vecteurs selon la revendication 1, dans lequel ledit premier plasmide comprend en outre des sites de clonage pour l'insertion d'une séquence d'acide nucléique.

13. Système de vecteurs selon la revendication 1, ledit premier plasmide comprenant un fragment ou des fragments de plasmide bactérien pBR322 qui code une résistance à l'ampicilline et également une origine de réplication de pBR322 qui permet audit vecteur d'être répliqué dans des cellules dans lesquelles pBR322 est capable d'être répliqué, dans lequel ledit vecteur est en outre modifié pour renfermer un insert entre la région précoce 4 (E4) et les séquences répétées inversées terminales droites, lequel vecteur présentant en outre une délétion de séquences d'E1 depuis la position 188 jusqu'à la séquence Af1II au niveau de la position 3533 ou proche de celle-ci, dans lequel ledit vecteur renferme des sites de clonage pour l'insertion d'un acide nucléique étranger.

14. Plasmide comprenant des séquences ADN d'adénovirus Ad5 de bp 19 (terminal du génome gauche) à bp 188 ; bp 1339 à bp 28133 ; et bp 30818 à bp 35934 (terminal du génome droit), dans lequel les terminaux du génome gauche et droit des séquences d'Ad5 sont joints de manière covalente, et comprenant en outre un fragment du plasmide pBR322 consistant en des nucléotides 375-1/4361-2064 insérés entre bp 188 et bp 1339 d'Ad5 et un site de restriction PacI positionné entre bp 28133 et bp 30818 d'Ad5.

15. Plasmide comprenant des séquences ADN d'adénovirus Ad5 de bp 19 (terminal du génome gauche) à bp 188 ; bp 1339 à bp 35934 (terminal du génome droit), dans lequel les terminaux du génome gauche et droit des séquences d'Ad5 sont joints de manière covalente, et comprenant en outre un fragment du plasmide pBR322 consistant en des nucléotides 375-1/4361-2064 insérés entre bp 188 et bp 1339 d'Ad5.

16. Plasmide comprenant des séquences ADN d'adénovirus Ad5 de bp 19 (terminal du génome gauche) à bp 188 ; bp 1339 à bp 27865 ; et bp 30995 à bp 35934 (terminal du génome droit), dans lequel les terminaux du génome gauche et droit des séquences d'Ad5 sont joints de manière covalente, et comprenant en outre un fragment du plasmide pBR322 consistant en des nucléotides 375-1/4361-2064 insérés entre bp 188 et bp 1339 d'Ad5 et un site de restriction PacI positionné entre bp 27865 et bp 30995 d'Ad5.

17. Système de vecteurs selon la revendication 1, dans lequel le deuxième plasmide est un plasmide navette E1 qui comprend ladite séquence dérivée de la région E1 d'Ad5, notamment un signal d'emballage.

18. Système de vecteurs selon la revendication 17, dans lequel un acide nucléique étranger qui comprend une phase de lecture ouverte est inséré dans ledit plasmide navette E1.

19. Système de vecteurs selon la revendication 18, dans lequel des séquences qui régulent l'expression de ladite phase de lecture ouverte sont en outre insérées dans ledit plasmide navette E1.

20. Système de vecteurs selon la revendication 17, dans lequel ledit plasmide navette E1 comprend une délétion pour la séquence dérivée de la région E1 d'Ad5 pour le fragment d'environ 3,19 kb entre le site SspI à 339bp et un site Af1II à 3533 bp, lequel plasmide comprend en outre un site de liaison sp1 synthétique et des sites de clonage pour l'insertion d'un acide nucléique étranger au sein de la délétion E1.

21. Système de vecteurs selon la revendication 17, dans lequel ledit plasmide navette E1 comprend une délétion dans la séquence dérivée de la région E1 d'Ad5 et comprend en outre un promoteur HCMV positionné au sein de la délétion E1 et des sites de clonage pour l'insertion d'un acide nucléique étranger positionné de manière adjacente au dit promoteur HCMV au sein de la délétion E1.

22. Système de vecteurs selon la revendication 17, dans lequel ledit plasmide navette E1 comprend une délétion dans la séquence dérivée de la région E1 d'Ad5 et qui comprend en outre un promoteur HCMV et un signal de polyadénylation SV40 tous deux positionnés au sein de la délétion E1 et de sites de clonage pour l'insertion d'un acide nucléique étranger positionné entre le promoteur HCMV et le signal de polyadénylation SV40.

23. Système de vecteurs selon la revendication 17, dans lequel ledit plasmide navette E1 comprend une délétion d'environ 2,88 kb dans la séquence dérivée de la région E1 d'Ad5 et qui comprend en outre des sites de clonage pour l'insertion d'un acide nucléique étranger positionné au sein de la délétion E1.

24. Système de vecteurs selon la revendication 17, dans lequel ledit plasmide navette E1 comprend une délétion dans la séquence dérivée de la région E1 d'Ad5 et qui comprend en outre un promoteur de l'actine béta et un signal de polyadénylation SV40 tous deux positionnés au sein de la délétion E1 et de sites de clonage pour l'insertion d'un acide nucléique étranger positionné entre le promoteur de l'actine béta et le signal de polyadénylation SV40.

25. Système de vecteurs selon la revendication 17, dans lequel ledit plasmide navette E1 comprend un signal d'emballage inséré entre la région précoce 4 (E4) et les séquences répétées inversées terminales droites.

26. Procédé in vitro d'introduction et d'expression d'un acide nucléique étranger dans une cellule hôte, comprenant les étapes consistant à :
(a) introduire
(i) un premier plasmide comprenant un génome d'adénovirus modifié qui comprend une modification au sein de la région précoce 1 (E1) dudit génome qui élimine le signal d'emballage dans ledit génome ; et
(ii) un plasmide navette E1 qui renferme une séquence dérivée d'une région E1 d'Ad, comprenant un signal d'encapsidation et une séquence d'acide nucléique insérée, dans une cellule hôte qui exprime des fonctions codées par la séquence E1 qui a été délétée du premier plasmide ; et
(b) isoler un génome viral recombinant dans lequel le premier plasmide s'est recombiné avec le plasmide navette E1 pour produire un génome viral modifié recombinant renfermant le signal d'encapsidation, la séquence d'acide nucléique insérée du plasmide navette E1 et des éléments du premier plasmide à l'exception de la modification au sein d'E1,
(c) introduire ledit génome viral modifié recombinant dans une cellule hôte, et
(d) exprimer les séquences codantes renfermées dans ledit génome viral modifié recombinant.

27. Procédé selon la revendication 26, dans lequel ledit premier plasmide comprend en outre un fragment ou des fragments d'un plasmide bactérien qui code une résistance antibiotique et comprend l'origine de réplication du plasmide et autres séquences nécessaires pour permettre au vecteur d'être répliqué dans des cellules dans lesquelles le plasmide est capable d'être répliqué.

28. Procédé selon la revendication 26 ou la revendication 27, dans lequel une séquence d'acide nucléique qui comprend une phase de lecture ouverte est en outre insérée dans ledit plasmide navette E1.

29. Procédé selon la revendication 28, dans lequel des séquences qui régulent l'expression de ladite phase de lecture ouverte sont en outre insérées dans le plasmide navette E1.

30. Procédé selon l'une quelconque des revendications 26 à 29, dans lequel ledit génome viral recombinant est emballé dans une particule virale recombinante qui est capable d'infecter des cellules qui peuvent être infectées par l'adénovirus à partir duquel le génome d'adénovirus modifié a été dérivé au départ.

31. Procédé selon l'une quelconque des revendications 26 à 30, dans lequel ledit génome d'adénovirus modifié a été dérivé au départ de l'adénovirus 5.

32. Procédé selon l'une quelconque des revendications 26 à 31, dans lequel ledit vecteur présentant une délétion E1 est introduit dans lesdites cellules hôtes avec une particule adénovirale présentant une région E1 intacte, dans lequel ladite particule adénovirale présentant une région E1 intacte est capable de complémenter la réplication dudit vecteur présentant une délétion E1.
